(19) Europäisches Patentamt
European Patent Office
Office européen des brevets

(11) Veröffentlichungsnummer : **0 378 197 B1**

# (12) EUROPÄISCHE PATENTSCHRIFT

(45) Veröffentlichungstag der Patentschrift : **29.03.95 Patentblatt 95/13**

(51) Int. Cl.$^6$ : **G01N 33/82**

(21) Anmeldenummer : **90100449.9**

(22) Anmeldetag : **10.01.90**

## (54) Vitamin-B12-Bestimmung.

(30) Priorität : **11.01.89 DE 3900650**

(43) Veröffentlichungstag der Anmeldung : **18.07.90 Patentblatt 90/29**

(45) Bekanntmachung des Hinweises auf die Patenterteilung : **29.03.95 Patentblatt 95/13**

(84) Benannte Vertragsstaaten : **AT BE CH DE DK ES FR GB GR IT LI LU NL SE**

(56) Entgegenhaltungen :
**EP-A- 0 378 203**
**EP-A- 0 378 204**
**JOURNAL OF EXPERIMENTAL MEDICINE, Band 188, Nr. 1, Mai 1988, New York, NY (US); R. CARMEL et al., Seiten 77-81**

(73) Patentinhaber : **BOEHRINGER MANNHEIM GMBH**
**D-68298 Mannheim (DE)**

(72) Erfinder : **Hoyle, Nicholas R., Dr.**
**Bräuhausstrasse 25**
**D-8132 Tutzing (DE)**
Erfinder : **Pappert Gunter, Dr.**
**Beringerweg 10**
**D-8132 Tutzing (DE)**
Erfinder : **Grol, Michael, Dr.**
**Possenhofener Strasse 22**
**D-8133 Feldafing (DE)**
Erfinder : **Hübner-Parajsz, Christa, Dr.**
**Marienstrasse 11**
**D-8132 Tutzing (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

EP 0 378 197 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Vitamin B12, oder Cobalamin, ist ein in Körperflüssigkeiten wie Vollblut, Plasma, Serum in niedriger Konzentration (ca. $10^{-14}$ mol/l) vorkommendes essentielles Vitamin, für das insbesondere die starke Bindung an die B12-Transportproteine (die Trans-Cobalamine) bemerkenswert ist. Die Erscheinungen von Vitamin B12-Mangel, der begründet sein kann in einer unzureichenden Vitaminzufuhr durch die Nahrung, durch Malabsorptions-Syndrome, einen genetisch induzierten Mangel eines oder mehrerer Trans-Cobalamine, oder durch die Gegenwart von Darmparasiten, wie z.B. dem Fischbandwurm (Diphyllobothria), kann sich in verschiedenen Erscheinungsbildern zeigen, die vom Alter des Individuums und der Dauer der Vitamin B12-Insuffizienz abhängen. Ein geringer Vitamin B12-Mangel bewirkt eine Verringerung der roten Blutkörperchen, wodurch weiter eine Reihe von Stoffwechselstörungen auftreten, und megoblastische Anämien. Bei Kindern wird das Nervensystem angegriffen, und in einigen Fällen kann Blindheit resultieren.

Die derzeit gebräuchlichen Methoden zur Bestimmung von Cobalaminen, insbesondere von Cyanocobalamin (Vitamin B12) in stark verdünnten wäßrigen Lösungen (wie z.B. dem Blutserum) basieren auf Verfahren unter Verwendung radioaktiver Markierungsstoffe, in denen Intrinsic-Factor (IF) als bindendes Reagenz verwendet wird. Die gebräuchlichen Techniken arbeiten unter Verwendung von $^{57}$Co-B12 als Marker unter Zugrundelegung eines kompetitiven Prinzips, bei dem freie und markierte Analyte um die Bindung mit dem IF kompetieren. Die Trennung von gebundenem und freiem Analyt (bound/free-Trennung) erfolgt dann aufgrund von Methoden wie z.B. unter Verwendung von Aktivkohle, festphasengebundenem IF oder magnetischer Trennung, bei der IF an paramagnetische Partikel gebunden ist (vgl. Brit. J. Haemat. 22 (1972) 21-31, Clin. Chem. 24 (1978) 460-466, Clin. Biochemistry 18 (1985) 261-266).

Vor der Bestimmung von Vitamin B12 in Körperflüssigkeiten ist es erforderlich, Vitamin B12 von seinen im Blut vorhandenen Bindeproteinen abzulösen. Dies erfolgt beispielsweise durch Hitzeeinwirkung oder durch Zerstörung des Bindeproteins im alkalischen Bereich (pH > 13,5) unter der Einwirkung des SH-Bindungen spaltenden Thiols Dithiothreitol (DTT) (Inkubation der Serumprobe mittels DTT im alkalischen Bereich). Durch Zugabe von organischen Stoffen, z.B. Aceton, oder durch Zugabe von kompetitiven, kreuzreagierenden Spezies, z.B. Cobinamid, kann diese Zerstörung verstärkt werden. Vorteilhaft wird bei der Bestimmung außerdem Alkalicyanid zugesetzt, um die Extraktionsfähigkeit des Vitamin B12 zu steigern, und um die Cobalamine in eine stabile und nachweisbare Form, nämlich das Cyanocobalamin, zu überführen.

Die Nachteile der bekannten Verfahren zur Bestimmung von Vitamin B12 sind insbesondere in der Verwendung von Intrinsic-Factor begründet. So werden falsche Ergebnisse beobachtet, wenn der verwendete Intrinsic-Factor nicht ausreichend rein ist (max. 5% Verunreinigung durch andere B12-Bindeproteine). Zahlreiche Proben enthalten offenbar Antikörper gegen IF, welche die IF-Bindungsfähigkeit für radiomarkiertes B12 blockieren. Dies kann zu niedrige Vitamin B12-Werte vortäuschen.

Aufgabe der vorliegenden Erfindung war es daher, ein Verfahren zur Bestimmung von Vitamin B12 bereitzustellen, mit dem auf die Verwendung von Intrinsic-Faktor verzichtet werden kann und damit die vorstehend genannten Nachteile vermieden werden können, und das auf rasche, einfache und reproduzierbare Weise eine genaue Bestimmung von Vitamin B12 im Serum ermöglicht.

Gegenstand der Erfindung ist daher ein Verfahren zur Bestimmung von Vitamin B12 durch Inkubation einer Probelösung mit mindestens zwei Rezeptoren $R_1$ und $R_2$, von denen $R_1$ die Bindung an die feste Phase vermittelt und $R_2$ markiert ist, Trennung der beiden Phasen und Messung der Markierung in einer der beiden Phasen, das dadurch gekennzeichnet ist, daß man als einen der beiden Rezeptoren $R_1$ oder $R_2$ einen Rezeptor verwendet, der einen spezifisch mit B12 bindefähigen, monoklonalen Antikörper, der für B12 eine Affinitätskonstante von mindestens 5 x $10^9$ l/mol hat, enthält und als anderen Rezeptor $R_2$ oder $R_1$ einen Rezeptor verwendet, der B12 oder ein Analogon davon enthält.

Das erfindungsgemäße Verfahren bedeutet einen entscheidenden Fortschritt für die klinische Diagnostik, da die Vitamin B12-Bestimmung einer der letzten Parameter war, für die kein immunologischer Test, unter Verwendung von immobilisierten monoklonalen Antikörpern kommerziell erhältlich war.

Für die erfindungsgemäße immunologische Bestimmungsmethode eignen sich im Prinzip alle gängigen Immuno-Assays, wie Radio-Immuno- Assay, Enzym-Immuno-Assay, Fluoreszenz-Immuno-Assay usw. Ferner sind sämtliche Verfahrensvarianten, wie kompetitiver Immuno-Assay, IEMA-Verfahren usw., anwendbar.

Zur Bestimmung von Vitamin B12 hat sich als besonders zweckmäßig ein kompetitiver Enzym-Immuno-Assay oder ein Verfahren nach dem IEMA-Prinzip erwiesen. Bei dem kompetitiven Enzym-Immuno-Assay konkurriert das zu bestimmende B12 mit einer bekannten Menge markiertem B12 um die Bindungsstellen des trägergebundenen monoklonalen Antikörpers. Das Testverfahren kann auch so durchgeführt werden, daß das zu bestimmende B12 und trägergebundenes B12 um im Unterschuß vorhandene Bindungsplätze des markierten monoklonalen Antikörpers konkurrieren. Der an das trägerfixierte B12 gebundene Anteil des markierten monoklonalen Antikörpers wird anhand der Markierung bestimmt. Diese Variante kann auch so abgewandelt werden, daß der monoklonale Antikörper nicht markiert eingesetzt wird. Der an das trägerfixierte B12 gebundene Antikörperanteil wird dann ermittelt, indem mit einem gegen den Fc-Teil des Antikörpers gerichteten Antikörper inkubiert und der gebundene markierte Anteil bestimmt wird. Bei dem IEMA-Verfahren wird markierter, monoklonaler Antikörper im Überschuß zugegeben. Der überschüssige, nicht an B12 gebundene markierte Antikörper wird mit Hilfe einer Hapten-Trägermatrix aus der Lösung entfernt. Die verschiedenen Varianten dieser Testmethoden sowie Einzelheiten zur Durchführung dieser Verfahren sind in der Literatur ausführlich beschrieben. Es sind jedoch auch andere immunologische Haptenbestimmungsverfahren zur B12-Bestimmung unter Verwendung der erfindungsgemäßen Antikörper möglich, so wie sie z.B. in den deutschen Anmeldungen DE-A 38 34 766 oder DE-A 38 22 750 beschrieben sind.

Erfindungsgemäß wird mindestens ein monoklonaler Antikörper eingesetzt, der spezifisch gegen Vitamin B12 gerichtet ist und eine Affinitätskonstante von $>5x10^9$ l/mol, vorzugsweise größer $10^{10}$ l/mol und besonders bevorzugt größer $5x10^{10}$ l/mol sowie eine Kreuzreaktivität gegen

Methylcobalamin und Cyanocobalamin von 100% gegen Cobinamid von < 0,05 %, gegen Purinylcobinamid von 1,1 %, gegen Cobryrinsäure-diamid von < 0,05 % gegen 2-Hydroxy-5,6-dimethylbenzimidazolylcobamid von 1,5 % und gegen (Carboxy(2-cyanamino-4,5-dimethylphenyl)amino)cobamid von 0,07 % aufweist.

Die monoklonalen Antikörper können als komplette Antikörper, chimäre Antikörper oder bivalente Antikörperfragmente eingesetzt werden.

Zur Bestimmung von Vitamin B12 wird die Probelösung daher mit mindestens zwei Rezeptoren $R_1$ und $R_2$ inkubiert.

Rezeptor $R_1$ vermittelt dabei die Bindung an die Festphase. Dazu kann der Rezeptor $R_1$ entweder direkt oder über einen Spacer an der Festphase gebunden sein oder aber in löslicher Form vorliegen und erst nach Durchführung der immunologischen Reaktion immobilisiert werden. Rezeptor $R_1$ enthält entweder einen spezifisch mit Vitamin B12 bindefähigen, monoklonalen Antikörper oder Vitamin B12 oder ein Analogon davon.

Die Bindung an den Träger (Immobilisierung) des Antikörpers bzw. von B12 erfolgt nach den dem Fachmann geläufigen Methoden durch adsorptive oder chemische Bindung oder durch Bindung über ein spezifisches Bindungspaar. In diesem Falle ist ein Partner des Bindungspaares immobilisiert, während der andere Partner chemisch an B12 bzw. den Antikörper gebunden ist. Über dieses Bindungspaar kann dann der Antikörper bzw. B12 vor oder während der immunologischen Bestimmungsreaktion immobilisiert werden. Beispiele für derartige Bindungspaare sind Biotin-Streptavidin/Avidin, Hapten-Antikörper, Antigen-Antikörper, Concavalin-Antikörper, Zucker-Lectin, Hapten-Bindeprotein.

Als Trägermaterialien zur Immobilisierung der erfindungsgemäßen Antikörper bzw. zur Immobilisierung von B12 können Materialien verwendet werden, wie z.B. Röhrchen (tubes), Mikrotiterplatten, Kugeln (beads) oder Mikrocarrier aus Kunststoffen, wie Polystyrol, Vinylpolymere, Polypropylen, Polycarbonat, Polysaccharide, Silicone, Gummi oder auch behandeltes Glas (vgl. z.B. E.T. Maggio, "Enzyme Immunoassay" CAC Press, Florida, 1980, insbesondere Seiten 175 bis 178; EP-A-063 064; Bioengineering 16 (1974), 997-1003; C.J. Sanderson und D.V. Wilson, Immunology 20 (1971), 1061-1065). Insbesondere wird als Trägermaterial ein mit Avidin oder Streptavidin beschichtetes Trägermaterial, insbesondere Polystyrol, vorzugsweise hergestellt wie in EP-A 0 269 092 beschrieben, verwendet.

Rezeptor $R_2$ enthält ebenfalls entweder Vitamin B12 oder ein Analogon davon oder einen spezifisch mit Vitamin B12 bindefähigen, monoklonalen Antikörper und ist markiert. Zur Markierung eignen sich die für die jeweilige Bestimmungsmethode üblichen Mittel. So werden bei einem Radio-Immuno-Assay Radioisotope, beispielsweise $^{57}Co$, zur Markierung verwendet. Für einen Enzym-Immuno-Assay sind sämtliche, hierfür üblicherweise eingesetzten Enzyme, beispielsweise Peroxidase oder β-Galactosidase, geeignet. Für einen Fluoreszenz-Immuno-Assay sind die üblichen fluoreszierenden Gruppen als Marker brauchbar. Einzelheiten dieser verschiedenen Testmethoden und Verfahrensvarianten sind dem Fachmann bekannt. In analoger Weise wie bei der Bindung an die Festphase kann auch die Bindung der Markierung an B12 bzw. den Antikörper über ein spezifisches Bindungspaar erfolgen.

Die Bindung des Antikörpers bzw. von B12 an einen der obengenannten Bindungspartner erfolgt nach den dem Fachmann geläufigen Methoden, beispielsweise über Carbodiimid und Hydroxysuccinimid.

Bei Markierung von B12 mit einem Enzym wird vorzugsweise ein B12-Konjugat der Formel (I)

$$B12\text{-}CO\text{-}NH \left( NH\text{-}R\text{-}CO\text{-}NH \right)_x N{=}GP \qquad (I)$$

verwendet, worin B12 den aus Cyanocobalamin (Vitamin B12) durch Abspaltung einer $-CONH_2$-Gruppe gebildeten Rest und R eine Verknüpfungsgruppierung (Spacer) bedeutet, und x 0 oder 1 ist, und GP den Rest eines glykosylgruppenhaltigen Markerenzyms darstellt, das über einen Glykosylrest an die -NH-N=-Gruppierung gebunden ist. In der Formel (I) steht die -CONH-Gruppierung vorzugsweise in d-Stellung des B12-Restes, und in erster Linie werden B12-d-CO-NH-N=GP, und insbesondere B12-d-CO-NH-NH-CO-$CH_2$ ( O-$CH_2$-$CH_2$ $)_3$

O-$CH_2$-CO-NH-N=GP, verwendet. Als Markierungsenzym (GP) wird vorzugsweise Peroxidase (POD) verwendet.

Die B12-Konjugate der Formel (I) sind Gegenstand der DE-A-3900648 (Titel: Neue Cobalamin-Säurehydrazide und davon abgeleitete Cobalamin-Konjugate) der gleichen Anmelderin mit dem gleichen Anmeldetag. Sie lassen sich durch Kupplung (Kondensation) der Cobalamin-Säurehydrazide der Formel

$$B12\text{-}CO\text{-}NH\text{-}NH\text{-}(R\text{-}CO\text{-}NH\text{-}NH)_x\text{-}H$$

(worin B12, R und x die vorstehend genannte Bedeutung besitzen), die ebenfalls Gegenstand der vorstehend genannten gleichzeitig eingereichten DE-A-3900648 sind, mit den OH-Gruppen von Glykosylresten der Glykoproteine nach deren Oxidation und Ausbildung der Hydrazongruppierung -NH-N=CH-Glykoprotein unter an sich bekannten Bedingungen herstellen.

In einer bevorzugten Ausführungsform des erfindungsgemäßen Verfahrens wird die Probelösung zur Ablösung des Vitamins B12 von den Bindeproteinen in herkömmlicher Weise vorbereitet, indem die Bindeproteine durch Zusatz eines SH-Gruppen spaltenden Thiols, Dithiothreitol (DTT) im alkalischen Bereich (pH >13,5), oder aber durch 30- bis 6ominütiges Kochen und nachfolgende Zentrifugation zerstört werden.

Vorzugsweise wird in dem erfindungsgemäßen Verfahren zur Vitamin B12-Bestimmung die Probenvorbereitung (Abspaltung des Bindeproteins) mit Liponsäure (Liponic acid, LA) oder einem Homologen davon der Formel (II)

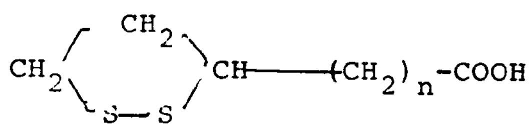

(II)

durchgeführt, worin n 1 bis 8, und insbesondere 3 bis 5 bedeutet, wobei die Liponsäure (Formel II, n=4) besonders bevorzugt ist.

Dieses Verfahren zur Probenvorbereitung ist Gegenstand der DE-A-3900649 (Titel: Verfahren zur Ablösung eines Analyten von seinem Bindeprotein) der gleichen Anmelderin mit dem gleichen Anmeldetag. Nach diesem Verfahren kann die Inkubation der Probe bei Raumtemperatur im alkalischen Bereich (pH-Wert 10 bis 14; unter vorzugsweiser Verwendung von Natriumhydroxid als alkalisches Medium, mit einer Konzentration von 0,05 bis 1 mmol/l) in weniger als 15 Minuten erfolgen.

Die Säure der Formel (II) wird dabei (für Liponsäure mit n=4 berechnet) vorzugsweise in einem Bereich von 1 bis 20 mg/ml, und insbesondere im Bereich von 4 bis 10 mg/ml eingesetzt.

Das erfindungsgemäße Verfahren liefert sehr genaue und reproduzierbare Werte, was vor allem darauf zurückzuführen ist, daß ein monoklonaler Antikörper gegen Vitamin B12 verwendet wird, der eine sehr hohe Affinitätskonstante gegenüber Vitamin B12 aufweist. Diese Antikörper sind ebenfalls Gegenstand der Erfindung. Derartig spezifische monoklonale Antikörper mit einer derartig hohen Affinitätskonstante sind bisher noch nicht bekannt.

Ein weiterer Gegenstand der Erfindung ist ein Verfahren zur Herstellung eines mit B12 spezifisch bindefähigen, monoklonalen Antikörpers, das dadurch gekennzeichnet ist, daß man Inzuchtmäuse mit Vitamin B12-d-Säure, an die über einen Spacer, insbesondere 1-Ethyl-3-(3-dimethylaminopropyl)-carbodiimid ein immunogenes Trägermaterial gekuppelt ist, immunisiert, B-Lymphocyten der immunisierten Tiere gewinnt und mit transformierenden Agentien mit Myelomzellen fusioniert, die so gebildeten Hybridzellen kloniert und kultiviert und den monoklonalen Antikörper aus diesen Zellen isoliert.

Zur Gewinnung der erfindungsgemäßen monoklonalen Antikörper wird B12 zunächst mit einem immunogenen Trägermaterial verknüpft. Als immunogene Trägermaterialien eignen sich alle üblicherweise für diesen Zweck verwendeten Stoffe, beispielsweise Albumine, wie Rinderserum-Albumin, Edestin usw. Die Verknüpfung von B12 mit dem Trägermaterial erfolgt nach an sich bekannten Methoden.

Anschließend werden Versuchstiere, beispielsweise Mäuse, mit dem immunogenen Konjugat immunisiert. Zur Immunisierung wird das Immunogen beispielsweise in Kombination mit dem Adjuvans in üblicher Weise verabreicht. Bevorzugt wird als Adjuvans komplettes oder inkomplettes Freundsches Adjuvans eingesetzt. Die Immunisierung erfolgt vorzugsweise über mehrere Monate mit mindestens vier Immunisierungen in vier- bis sechswöchigem Abstand (Injektion intraperitoneal).

Aus so immunisierten Tieren werden B-Lymphozyten gewonnen, die mit einer permanenten Myelomzellinie fusioniert werden. Die Fusionierung erfolgt nach dem bekannten Verfahren von Köhler und Milstein (Nature 256, 1975, Seiten 495 bis 497). Die hierbei gebildeten Primärkulturen von Hybridzellen werden

in üblicher Weise, z.B. unter Verwendung eines handelsüblichen Zellsorters oder durch "limiting dilution", kloniert. Es werden jeweils die Kulturen weiterverarbeitet, die in einem geeigneten Testverfahren, beispielsweise einem Enzym-Immuno-Assay (ELISA-Verfahren), positiv gegen B12 sind und die genannten Kreuzreaktivitäten zeigen. Man erhält so mehrere Hybridoma-Zellinien, die die erfindungsgemäßen monoklonalen Antikörper produzieren. Nach bekannten Methoden können diese Zellinien kultiviert und die von ihnen produzierten monoklonalen Antikörper isoliert werden.

Auf diese Weise können die erfindungsgemäß verwendeten Antikörper erhalten werden, insbesondere Antikörper mit einer Affinitätskonstante von > $5x10^9$ l/mol, vorzugsweise größer $10^{10}$ l/mol, besonders bevorzugt größer $5x10^{10}$ l/mol sowie eine Kreuzreaktivität gegen Methylcobalamin und Cyanocobalamin von 100 % gegen Cobinamid von< 0,05 %, gegen Purinylcobinamid von 1,1 %, gegen Cobryrinsäurediamid von < 0,05 % gegen 2-Hydroxy-5,6-dimethylbenzimidazolylcobamid von 1,5 % und gegen (Carboxy(2-cyanamino4,5-dimethylphenyl)amino)cobamid von 0,07 % aufweist. Antikörper, die eine so hohe Spezifität zeigen, werden z.B. von den auf diese Weise gewonnene Zellinien ECACC 88101301 und ECACC 88101302 produziert.

Die Zellinien sind unter der jeweils angegebenen Nummer bei der Hinterlegungsstelle ECACC (European Collection of Animal Cell Cultures, Porton Down, GB) hinterlegt.

Die so gewonnenen monoklonalen Antikörper zeichnen sich dadurch aus, daß sie eine sehr hohe Affinität (Affinitätskonstante größer $5x10^{-9}$) für B12 und die genannten Kreuzreaktivitäten besitzen. Vorzugsweise liegt die Affinität der monoklonalen Antikörper über $10^{10}$ l/mol, besonders bevorzugt über $5x10^{10}$ l/mol.

Die erfindungsgemäßen monoklonalen Antikörper eignen sich hervorragend dazu, in einer Probe, beispielsweise Serum oder Plasma, das B12 spezifisch zu bestimmen. Für diese Bestimmungsverfahren können die monoklonalen Antikörper als solche chimäre Antikörper oder Fragmente hiervon, welche die entsprechenden immunologischen Eigenschaften aufweisen, beispielsweise Fab-Fragmente, verwendet werden. Unter dem Begriff "monoklonale Antikörper" werden daher sowohl vollständige Antikörper als auch die Fragmente verstanden.

Die nachfolgenden Beispiele sollen die Erfindung näher erläutern, ohne sie darauf zu beschränken. Unter Raumtemperatur (RT) wird eine Temperatur von 25°C ± 2°C verstanden. Prozentangaben beziehen sich auf Gewichtsprozent.

Fig. 1 zeigt eine Standardkurve für eine Vitamin B12-Bestimmung nach Beispiel 4 mit verschiedenen MAK-Konzentrationen:

Kurve 1: 85 ng/ml MAK
Kurve 2: 90 ng/ml MAK
Kurve 3: 95 ng/ml MAK
Kurve 4: 100 ng/ml MAK.

Fig. 2 zeigt einen Vergleich einer Bestimmung nach Beispiel 4 (Kurve 2) mit einer Bestimmung unter Verwendung von polyklonalen Antikörpern (Kurve 1).

**Beispiel 1**

Gewinnung von monoklonalen Antikörpern gegen Vitamin B12

Herstellung des Immunogens

Vitamin B12-d-Säure (hergestellt nach JACS 102 (1980) 2215) wird über 1-Ethyl-3-(3-dimethylaminopropyl)-Carbodiimid (EDC) an Edestin gekuppelt.

Immunisierung von Mäusen mit Vitamin B12-Konjugat

Balb/c-Mäuse, 8 bis 12 Wochen alt, wurden mit 100 µg Immunogen in komplettem Freundschen Adjuvans intraperitoneal erstimmunisiert. Nach sechs Wochen wurden drei weitere Immunisierungen in vierwöchigen Abständen durchgeführt. Dabei wurden 100 µg Immunogen in inkomplettem Freundschen Adjuvans intraperitoneal verabreicht. 4 Tage, 3 Tage und 2 Tage vor der Fusion wurde noch einmal mit 100 µg Immunogen in vitro immunisiert.

Fusion

Milzzellen einer immunisierten Maus wurden mit P3x63Ag8-653 Myelomzellen (ATCC-CRL 8375) im Verhältnis 1:5 gemischt und zentrifugiert (10 Minuten, 300 g, 4°C). Die Zellen wurden noch einmal mit BSS (Balanced Salt Solution)-Puffer gewaschen und bei 400 g in einem 50 ml Spitzröhrchen zentrifugiert. Der Überstand wurde abgekippt, das Zellsediment aufgelockert, 1 ml PEG (MG 4000, Merck) dazugegeben und durchpipettiert. Nach einer Minute im Wasserbad wurden bei Raumtemperatur 5 ml RPMI 1640-Medium (RPMI = Rosewell Parker Memory Institut) ohne FKS (fötales Kälberserum) in einem Zeitraum von 4 bis 5 Minuten zugetropft, durchgemischt, auf 50 ml mit Medium aufgefüllt und anschließend 10 Minuten bei 400 g, 4°C zentrifugiert. Die sedimentierten Zellen wurden in RPMI 1640-Medium + 10 % FKS aufgenommen und je $5x10^4$ bis $1x10^5$ Milzzellen oder $5x10^4$ Peritoneal-Exudat-Zellen als "Futterzellen" zugefügt. Am nächsten Tag wurde Hypoxanthin-Azaserin-Selektionsmedium (100 mmol/l Hypoxanthin, 1 µg/ml Azaserin) zugegeben.

Circa 7 bis 10 Tage nach der Fusion waren bereits viele Klone sichtbar. Der Überstand der Primärkulturen wurde nach einem in Beispiel 2 beschriebenen ELISA-Verfahren getestet. Primärkulturen, welche die gewünschte Kreuzreaktion zeigten, wurden mittels FACS (Fluorescence activated cell sorter) in 96-well-Zellkulturplatten kloniert. Als "Futterzellen" wurden $1x10^4$ Peritoneal-Exudat-Zellen oder $2x10^4$ Milzzellen pro "well" zugegeben. Auf diese Weise konnten beispielsweise die beiden Hybridoma-Zellinien

ECACC 88101301 und
ECACC 88101302

isoliert werden, die bei der Hinterlegungsstelle ECACC unter der jeweils angegebenen Hinterlegungsnummer hinterlegt worden sind.

Induktion von Ascites

$5x10^6$ Hybridzellen wurden i.p. in ein- bis zweimal mit 0,5 ml Pristan vorbehandelte Mäuse gespritzt. Ascites mit einer IgG-Konzentration von 5 bis 20 mg/ml konnte 1 bis 3 Wochen danach gewonnen werden. Hieraus können in üblicher Weise die Antikörper isoliert werden. Diese monoklonalen Antikörper sind spezifisch gegen Vitamin B12 gerichtet und zeigen die gewünschte Kreuzreaktivität. Die monoklonalen Antikörper werden mit MAK 1 (von ECACC 88101301) bzw. MAK 2 (von ECACC 88101302) bezeichnet.

**Beispiel 2**

Screening-Test auf Antikörper gegen Vitamin B12

Um die Anwesenheit und Spezifität von Antikörpern gegen Vitamin B12 im Serum immunisierter Mäuse oder in dem Kulturüberstand der Hybridzellen oder in Ascites zu erkennen, wird ein ELISA-Verfahren als Testprinzip verwendet: Mikrotiterplatten werden mit 1 µg/ml B12-Konjugat (B12-d-Säure gekoppelt an Rinderserumalbumin über EDC) Beschichtungspuffer (0,2 mol/l Natriumcarbonat/-Bicarbonat, pH 9,3 bis 9,5) bei 37°C eine Stunde lang beschichtet. Es wird 10 Minuten mit 0,9 % Natriumchloridlösung und 1 % Albuminlösung nachbehandelt. Anschließend wird mit 0,9 % Natriumchloridlösung gewaschen. Danach wird bei 37°C eine Stunde mit 100 µl Probe inkubiert und erneut mit 0,9 % Natriumchloridlösung gewaschen. Um die Kreuzreaktion zu prüfen, werden der Probelösung 50, 500 und 5000 µg/ml des zu prüfenden Vitamin B12-Derivats zugesetzt. Eine Erniedrigung des Meßsignals in Gegenwart der Derivate bedeutet Kreuzreaktion. Es folgt eine weitere Inkubation (eine Stunde, 37°C) mit 450 U/ml eines Schaf-Fab-anti-Maus-Fcγ-Peroxidase-Konjugats. Nach einem erneuten Waschschritt mit 0,9 % Natriumchlorid wird die Peroxidaseaktivität in üblicher Weise bestimmt (beispielsweise mit 2,2'-Azino-di-[3-ethylbenzthiazolin-sulfonat(6)] (ABTS®), 30 Minuten bei Raumtemperatur, abgelesen wird die Extinktionsdifferenz, ΔmE, bei 422 nm).

**Beispiel 3**

Bestimmung der Kreuzreaktion

Der Test wird, wie im Beispiel 2 beschrieben, durchgeführt.

Zu dem monoklonalen Antikörper wird jeweils das auf Kreuzreaktion zu prüfende Antigen in steigender Konzentration (50 µg, 500 µg, 5000 µg/ml) zugegeben. Die Kreuzreaktion wird anschließend nach folgender Formel berechnet:

$$\%\ \text{Kreuzreaktion} = \frac{\text{C (Vitamin B12)}}{\text{C (kreuzreagierendes Antigen)}} \times 100$$

C = Konzentration des Antigens, die zur Erreichung von 50 % des max. Signals erforderlich ist.

In der folgenden Tabelle sind die ermittelten Werte, die identisch für die monoklonalen Antikörper MAK 1 und MAK 2 sind, zusammengestellt.

| kreuzreagierendes Antigen | Kreuzreaktion |
|---|---|
| Methyl-cobalamin | 100 |
| Cyano-cobalamin | 100 |
| Cobinamid | <0,05 |
| Purinylcobinamid | 1,1 |
| Cobryinsäure-diamid | <0,05 |
| 2-Hydroxy-5,6-dimethyl-benzimidazolylcobamid | 1,5 |
| (Carboxy(2-cyanamino-4,5-dimethylphenyl)aminocobamid | 0,07 |

**Beispiel 4**

Bestimmung von Vitamin B12

a) Probenvorbereitung

250 µl Humanserum werden mit 125 µl Ablösereagenz (bestehend aus 8 mg/ml Liponsäure, 1 mg/ml Kaliumcyanid, gelöst in 0,5 mol/l NaOH) gemischt und 15 Minuten bei Raumtemperatur inkubiert. Anschließend werden 125 µl, 200 mmol/l Phosphatpuffer, pH 4,1 zugegeben.

b) Reagenzien:

Polystyrolröhrchen, beschichtet mit Thermo-RSA Streptavidin (hergestellt nach EP-A 0269092)

Reagenz 1

95 ng/ml biotinylierter MAK 1 oder MAK 2 (Biotinylierung nach JACS 100 (1978) 3585 bis 3590) 40 mmol/l Phosphatpuffer, pH 7,2,

Reagenz 2

B12-d-CO-NH-NH-CO-CH2-(-O-CH2-CH2-)$_3$-O-CH2-CO-NH-N=POD) (Aktivität ca. 60 mU/ml)

40 mmol/l Phosphatpuffer pH 7,2

Reagenz 3

100 mmol/l Phos hat-Citratpuffer, pH 4,4

1,9 mmol/l ABTS®

3,2 mmol/l Natriumperborat

c) Durchführung der Bestimmung

Zur Durchführung der Bestimmung werden 200 µl vorbehandelte Probe mit 800 µl Reagenz 1 in ein Streptavidin-tube gegeben und 60 Minuten bei Raumtemperatur inkubiert. Anschließend wird mit Waschlösung gewaschen und 1000 µl Reagenz 2 zugegeben und 30 Minuten bei Raumtemperatur inkubiert. Es wird mit Waschlösung gewaschen und 1000 µl Reagenz 3 zugegeben, für 30 Minuten bei Raumtemperatur inkubiert und die gebildete Farbe als Maß für den Vitamin B12-Gehalt bei 422 nm gemessen.

d) Analoge Ergebnisse werden erhalten, wenn anstelle von biotinyliertem komplettem MAK 1 biotinylierte Fab-Fragmente eingesetzt werden. Fab-Fragmente werden folgendermaßen hergestellt:

Mit MAK 1 wird, wie in Biochem. J. 73 (1959) 119 bis 126, eine Papainspaltung durchgeführt. Die hierbei entstandenen Fab-Fragmente werden mittels Gelfiltration über Sephadex® G 100 und Ionenaustauschchromatographie über DEAE Cellulose nach Meth. in Enzymology 73 (1981) 418 bis 459 abgetrennt.

**Beispiel 5**

Vergleich mit einem bekannten Radioimmunoassay für B12

Als Standard wurde Cyanocobalamin in 40 mmol/l Phosphatpuffer, pH 7,2 mit 0,9 % Natriumchlorid, 0,9 % Crotein C und 0,1 % Kaliumcyanid verwendet. Zum Vergleich wurde der von Becton Dickinson vertriebene

Test (Simultaneous No Boil SNB-B12/Folat-Radioassay) verwendet. Bei diesem Test wird immobilisierter Intrinsic Factor und radioaktiv markiertes B12 ($^{57}$Co B12) verwendet. Zur Probenvorbereitung wird bei diesem Test Dithiothreitol (DTT) in alkalischer Lösung verwendet. Die Korrelation zwischen diesem Radioimmunoassay und dem erfindungsgemäßen Verfahren ist im Bereich einer Vitamin B12-Konzentration zwischen 100 und 1400 pg/ml>0,98.

**Beispiel 6**

Vitamin B12-Bestimmung mit polyklonalem Antikörper gegen B12 (Vergleichsbeispiel)

a) Gewinnung des Antiserums

10 Schafe werden mit dem im Beispiel 1 beschriebenen Immunogen (0,5 ng/ml in komplettem Freundschen Adjuvans) in vierwöchigem Abstand über 6 Monate immunisiert. Anschließend wird das Antiserum gewonnen und affinitätschromatographisch gereinigt.

b) Herstellung von biotinylierten Fab-Fragmenten des polyklonalen Antikörpers gegen B12 (Fab-Biotin)

Mit den polyklonalen Antikörpern wird wie in Biochem. J. 73 (1959) 119-126 beschrieben, eine Papainspaltung durchgeführt. Die hierbei entstandenen Fragmente werden mittels Gelfiltration über Sephadex® G100 und Ionenaustauscher-Chromatographie über DEAE-Cellulose nach Meth. in Enzymology 73 (1981) 418-459 abgetrennt.

Die Biotinylierung erfolgt wie in JACS 100 (1978) 3585-3590 beschrieben.

c) Durchführung der Bestimmung

Die Durchführung der Bestimmung erfolgt, wie im Beispiel 4 beschrieben, wobei anstelle von 95 ng/ml biotinyliertem MAK1 95 ng/ml Fab-Biotin eingesetzt werden.

Fig. 2 zeigt den Vergleich zwischen einer B12-Bestimmung über polyklonale und monoklonale Antikörper. Danach zeigt sich, daß mit den erfindungsgemäßen monoklonalen antikörpern eine wesentlich steilere Eichkurve erhalten wird.

## Patentansprüche

1. Verfahren zur Bestimmung von Vitamin B12 durch Inkubation einer Probelösung mit mindestens zwei Rezeptoren $R_1$ und $R_2$, von denen $R_1$ die Bindung an die feste Phase vermittelt und $R_2$ markiert ist, Trennung der beiden Phasen und Messung der Markierung in einer der beiden Phasen,
**dadurch gekennzeichnet,** daß, man als einen der beiden Rezeptoren $R_1$ oder $R_2$ einen Rezeptor verwendet, der einen spezifisch mit B12 bindefähigen, monoklonalen Antikörper, der für B12 eine Affinitätskonstante von mindestens 5 x $10^9$ l/mol hat, enthält und als anderen Rezeptor $R_2$ oder $R_1$ einen Rezeptor verwendet, der B12 oder ein Analogon davon enthält.

2. Verfahren nach Anspruch 1,
**dadurch gekennzeichnet,** daß, man als spezifisch mit B12 bindefähigen monoklonalen Antikörper einen Antikörper verwendet, der eine Kreuzreaktivität gegen Methylcobalamin und Cyanocobalamin von 100 %; gegen Cobinamid von < 0,05 %; gegen Purinylcobinamid von 1,1 %; gegen Cobryrinsäure-diamid von < 0,05 %; gegen 2-Hydroxy-5,6-dimethylbenzimidazolyl-cobamid von 1,5 % und gegen (Carboxy(2-cyanamino-4,5-dimethylphenyl)-amino)-cobamid von 0,07 % aufweist.

3. Verfahren nach Anspruch 1 oder 2,
**dadurch gekennzeichnet,** daß, man als monoklonalen Antikörper einen von der Zellinie ECACC 88101301 oder ECACC 88101302 produzierten Antikörper verwendet.

4. Verfahren nach einem der vorhergehenden Ansprüche,
**dadurch gekennzeichnet**, daß als Rezeptor $R_2$ ein B12-Konjugat der folgenden Formel:

$$B12\text{-}CO\text{-}NH\text{-}(\text{-}NH\text{-}R\text{-}CO\text{-}NH\text{-})_x\text{-}N{=}GP$$

verwendet wird, worin B12 den aus Cyanocobalamin durch Abspaltung einer $CONH_2$-Gruppe gebildeten Rest und R einen Spacer bedeutet, x 0 oder 1 ist und GP den Rest eines glykosylgruppenhaltigen Markerenzyms darstellt, das über einen Glykosylrest an die NH-N=Gruppierung gebunden ist.

5. Verfahren nach einem der vorhergehenden Ansprüche,

**dadurch gekennzeichnet**, daß man die Probelösung vor Inkubation mit den beiden Rezeptoren $R_1$ und $R_2$ durch Zugabe einer Säure der allgemeinen Formel (I):

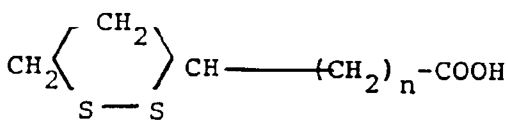 (I)

vorbehandelt, worin n eine ganze Zahl von 1 bis 8 bedeutet.

6. Verfahren nach Anspruch 5, **dadurch gekennzeichnet**, daß man zur Vorbehandlung Liponsäure verwendet.

7. Verfahren zur Herstellung eines mit B12 spezifisch bindefähigen, monoklonalen Antikörpers, **dadurch gekennzeichnet**, daß man Inzuchtmäuse mit Vitamin B12-d-Säure, an die über einen Spacer, insbesondere 1-Ethyl-3-(3-dimethylaminopropyl)-carbodiimid ein immunogenes Trägermaterial gekuppelt ist, immunisiert, B-Lymphocyten der immunisierten Tiere gewinnt und mit transformierenden Agentien mit Myelomzellen fusioniert, die so gebildeten Hybridzellen kloniert und kultiviert und den monoklonalen Antikörper aus diesen Zellen isoliert.

8. Zellinie ECACC 88101301.

9. Zellinie ECACC 88101302.

10. Reagenz zur Bestimmung von B12, enthaltend einen die Bindung an die Festphase vermittelnden Rezeptor $R_1$ und einen markierten Rezeptor $R_2$, **dadurch gekennzeichnet**, daß es als einen der beiden Rezeptoren $R_1$ oder $R_2$ einen Rezeptor, der einen spezifisch mit B12 bindefähigen monoklonalen Antikörper, der für B12 eine Affinitätskonstante von mindestens $5x10^9$ l/mol aufweist, enthält und als anderen Rezeptor $R_2$ oder $R_1$ einen Rezeptor enthält, der B12 oder ein Analogon davon enthält sowie gegebenenfalls ein Nachweissystem für die Markierung.

11. Reagenz nach Anspruch 10, **dadurch gekennzeichnet**, daß es als Festphase eine mit Avidin oder Streptavidin beschichtete Matrix, als Rezeptor $R_1$ ein Konjugat aus Biotin und einem mit B12 spezifisch bindefähigen, monoklonalen Antikörper, als $R_2$ eine Verbindung der Formel B12-CO-NH ( NH-R-CO-NH ) $_x$ N=GP, worin B12 den aus Cyanocobalamin durch Abspaltung einer $CONH_2$-Gruppe gebildeten Rest, R einen Spacer, x 0 oder 1 und GP den Rest eines glykosylgruppenhaltigen Markerenzyms darstellt, das über einen Glykosylrest an die NH-N=-Gruppierung gebunden ist sowie ein für das Markerenzym geeignetes Nachweissystem enthält.

12. Reagenz nach einem der Ansprüche 10 oder 11, **dadurch gekennzeichnet**, daß es zusätzlich eine Verbindung der Formel

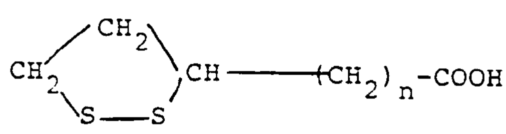 (I)

worin n 1 bis 8 bedeutet, enthält.

## Claims

1. Method for the determination of vitamin B12 by incubation of a sample solution with at least two receptors $R_1$ and $R_2$, of which $R_1$ mediates the binding to the solid phase and $R_2$ is labelled, separation of the two phases and measurement of the label in one of the two phases, **wherein** a receptor is used as one of the receptors $R_1$ or $R_2$ which contains a monoclonal antibody capable of specific binding to B12 that has an affinity constant of at least $5 \times 10^9$ l/mol and a receptor is used as the other receptor $R_1$ or $R_2$ which contains

B12 or an analogue thereof.

2. Method as claimed in claim 1, **wherein** an antibody is used as the monoclonal antibody capable of specific binding to B12 which has a cross-reactivity with methylcobalamin and cyanocobalamin of 100 %; with cobinamide of < 0.05 %; with purinylcobinamide of 1.1 %; with cobyrinic aciddiamide of < 0.05 %; with 2-hydroxy-5,6-dimethylbenzimidazolyl-cobamide of 1.5 % and with (carboxy(2-cyanamino-4,5-dimethylphenyl)-amino)-cobamide of 0.07 %.

3. Method as claimed in claims 1 or 2, **wherein** one of the antibodies produced by the cell line ECACC 88101301 or ECACC 88101302 is used as the monoclonal antibody.

4. Method as claimed in one of the previous claims, **wherein** a B12 conjugate of the following formula is used as receptor $R_2$:

$$B12-CO-NH-(-NH-R-CO-NH-)_x-N=GP$$

wherein B12 denotes the residue formed by cleavage of a $CONH_2$ group from cyanocobalamin and R denotes a spacer, x is 0 or 1 and GP represents a marker enzyme residue containing glycosyl groups which is bound via a glycosyl residue to the NH-N= group.

5. Method as claimed in one of the previous claims, **wherein** before incubation with the two receptors $R_1$ and $R_2$, the sample solution is pretreated by addition of an acid of the general formula (I):

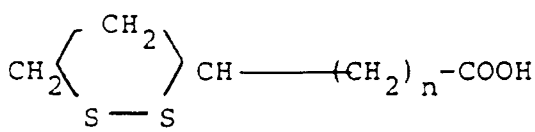

(I)

wherein n denotes a whole number from 1 to 8.

6. Method as claimed in claim 5, **wherein** lipoic acid is used for the pre-treatment.

7. Process for the production of a monoclonal antibody capable of specific binding to B12, **wherein** inbred mice are immunized with vitamin B12-d-acid to which an immunogenic carrier material is coupled via a spacer in particular 1-ethyl-3-(3-dimethylaminopropyl)-carbodiimide, B-lymphocytes are isolated from the immunized animals and fused with myeloma cells using transforming agents, the hybrid cells which form are cloned and cultured and the monoclonal antibodies are isolated from these cells.

8. Cell line ECACC 88101301.

9. Cell line ECACC 88101302.

10. Reagent for the determination of B12 containing a receptor $R_1$ which mediates the binding to the solid phase and a labelled receptor $R_2$, **wherein** it contains a receptor as one of the two receptors $R_1$ or $R_2$ which contains a monoclonal antibody capable of specific binding to B12 that has an affinity constant of at least 5 x $10^9$ l/mol for B12 and contains a receptor as the other receptor $R_2$ or $R_1$ which contains B12 or an analogue thereof as well as, if desired, a detection system for the label.

11. Reagent as claimed in claim 10, **wherein** it contains a matrix coated with avidin or streptavidin as the solid phase, a conjugate of biotin and a monoclonal antibody capable of specific binding to B12 as $R_1$, a compound of the formula $B12-CO-NH-(-NH-R-CO-NH-)_x-N=GP$ as $R_2$, wherein B12 denotes the residue formed by cleavage of a $CONH_2$ group from cyanocobalamin, R denotes a spacer, x is 0 or 1 and GP represents a marker enzyme residue containing glycosyl groups which is bound via a glycosyl residue to the NH-N= group as well as a suitable detection system for the marker enzyme.

12. Reagent as claimed in one of the claims 10 or 11, **wherein** it contains in addition a compound of the formula

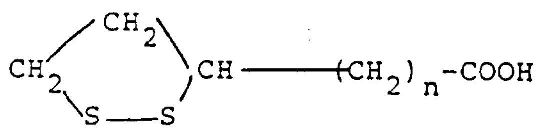

(I)

wherein n denotes 1 to 8.

## Revendications

1. Procédé de détermination de la vitamine B12 par incubation d'une solution échantillon avec au moins deux récepteurs $R_1$ et $R_2$ parmi lesquels $R_1$ permet la liaison avec la phase solide et $R_2$ est marqué, séparation des deux phases et mesure du marqueur dans l'une des deux phases, caractérisé en ce que l'on utilise comme l'un des deux récepteurs $R_1$ ou $R_2$ un récepteur qui contient un anticorps monoclonal qui peut se lier spécifiquement à B12, qui a pour B12 une constante d'affinité d'au moins 5 x $10^9$ l/mol, et comme autre récepteur $R_2$ ou $R_1$ un récepteur qui contient B12 ou un analogue de celle-ci.

2. Procédé selon la revendication 1, caractérisé en ce que l'on utilise comme anticorps monoclonal capable de se lier spécifiquement à B12 un anticorps qui présente une réactivité croisée vis à vis de la méthylcobalamine et de la cyanocobalamine de 100%, vis à vis du cobinamide < 0,05%, vis à vis du purinylcobinamide de 1,1%, vis à vis du diamide d'acide cobryrique < 0,05%, vis à vis du 2-hydroxy-5,6-diméthylbenzimidazolyl-cobamide de 1,5% et vis à vis du (carboxy(2-cyanamido-4,5-diméthylphényl)amino)cobamide de 0,07%.

3. Procédé selon la revendication 1 ou 2, caractérisé en ce que l'on utilise comme anticorps monoclonal un anticorps produit par la lignée cellulaire ECACC 88101301 ou ECACC 88101302.

4. Procédé selon l'une des revendications précédentes, caractérisé en ce que l'on utilise comme récepteur $R_2$ un conjugué de B12 de formule suivante:

$$B12-CO-NH-(-NH-R-CO-NH-)_x-N{=}GP$$

dans laquelle B12 représente le reste formé à partir de la cyanocobalamine par clivage d'un groupe $CONH_2$ et R représente un espaceur, x est égal à 0 ou 1 et GP représente le reste d'une enzyme de marquage contenant des groupes glycosyle qui est liée au groupement NH-N= par l'intermédiaire d'un reste glycosyle.

5. Procédé selon l'une des revendications précédentes, caractérisé en ce que, avant l'incubation avec les deux récepteurs $R_1$ et $R_2$ , on prétraite la solution échantillon par addition d'un acide de formule générale (I):

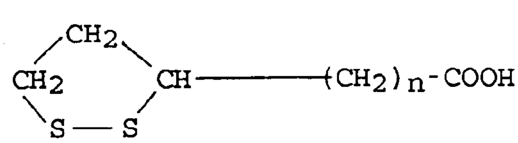

(I)

dans laquelle n représente un nombre entier de 1 à 8.

6. Procédé selon la revendication 5, caractérisé en ce que l'on utilise l'acide liponique pour le prétraitement.

7. Procédé de préparation d'un anticorps monoclonal capable de se lier spécifiquement à B12, caractérisé en ce que l'on immunise des souris d'élevage avec l'acide vitaminique B12-d auquel est couplé un matériau support immunogène par l'intermédiaire d'un espaceur, en particulier le 1-éthyl-3-(3-diméthylaminopropyl)carbodiimide, on obtient des lymphocytes B des animaux immunisés et on les fusionne avec des cellules de myélome avec des agents transformants, on clone les cellules hybrides ainsi formées et on les cultive, et on isole l'anticorps monoclonal à partir de ces cellules.

**8.** Lignée cellulaire ECACC 88101301.

**9.** Lignée cellulaire ECACC 88101302.

**10.** Réactif pour la détermination de B12, contenant un récepteur $R_1$ permettant la liaison à la phase solide et un récepteur marqué $R_2$, caractérisé en ce qu'il contient comme l'un des deux récepteurs $R_1$ ou $R_2$ un récepteur qui contient un anticorps monoclonal capable de se lier spécifiquement à B12 qui présente pour B12 une constante d'affinité d'au moins 5 x $10^9$ l/mol et comme autre récepteur $R_2$ ou $R_1$ un récepteur qui contient B12 ou un analogue de celle-ci et éventuellement un système de mise en évidence pour le marqueur.

**11.** Réactif selon la revendication 10, caractérisé en ce qu'il contient comme phase solide une matrice recouverte d'avidine ou de streptavidine, comme récepteur $R_1$ un conjugué de biotine et d'un anticorps monoclonal capable de se lier spécifiquement à B12, comme $R_2$ un composé de formule

$$B12\text{-}CO\text{-}NH\text{—}(\text{—}NH\text{-}R\text{-}CO\text{-}NH)_x N{=}GP,$$

dans lequel B12 représente le reste formé à partir de la cyanocobalamine par clivage d'un groupe $CONH_2$, R représente un espaceur, x représente 0 ou 1 et GP représente le reste d'une enzyme de marquage contenant des groupes glycosyle qui est liée au groupement NH-N= par l'intermédiaire d'un reste glycosyle et un système de mise en évidence approprié pour l'enzyme de marquage.

**12.** Réactif selon l'une des revendications 10 ou 11, caractérisé en ce qu'il contient en outre un composé de formule

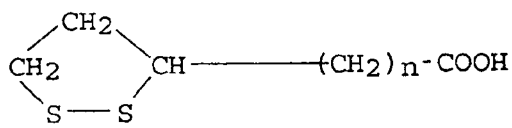

(I)

dans laquelle n représente un nombre de 1 à 8.

FIG.1

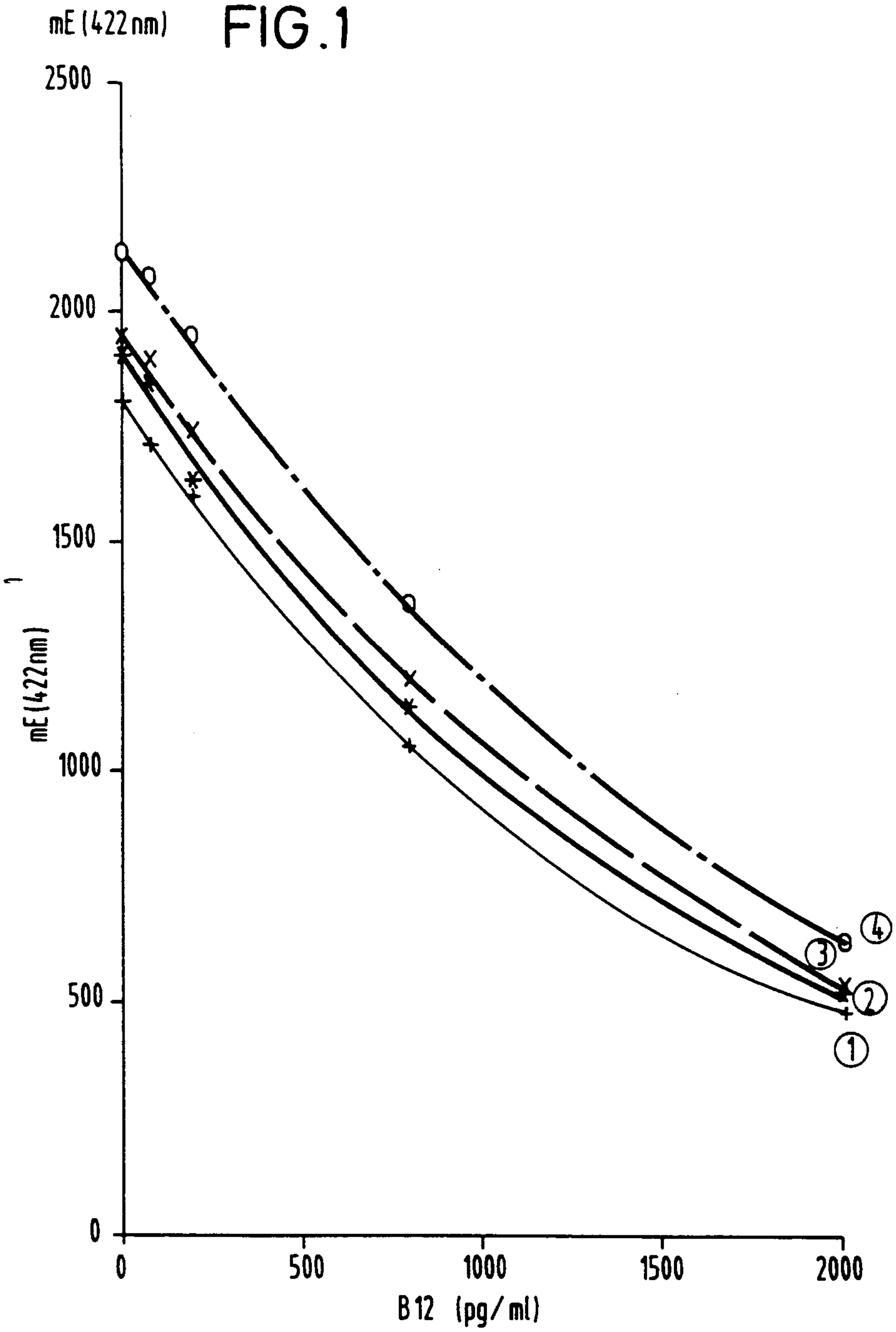
mE(422nm)
2500
2000
1500
1000
500
0
mE(422nm)
0
500
1000
1500
2000
B12 (pg/ml)
④
③
②
①

FIG. 2

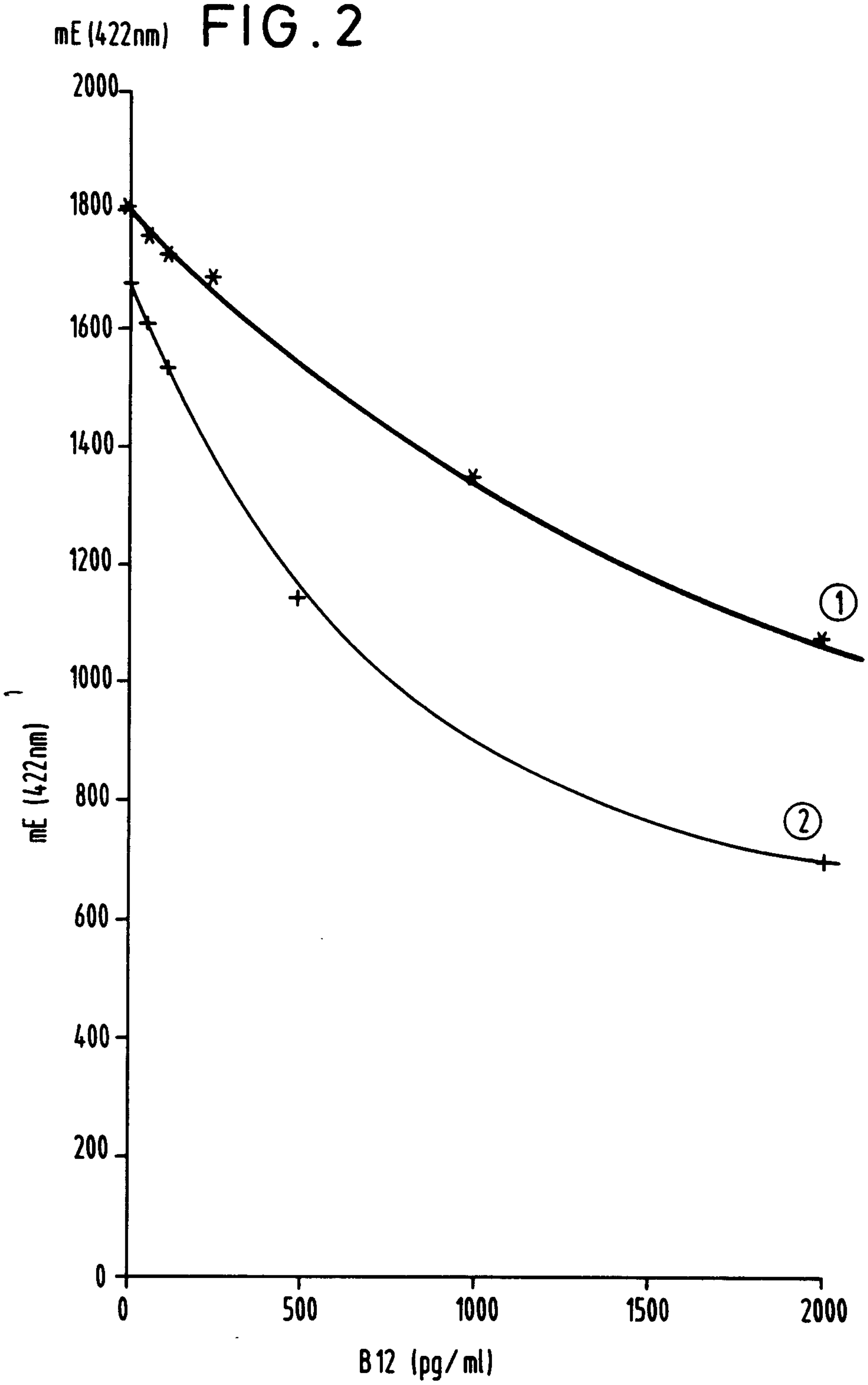
mE (422nm)
2000
1800
1600
1400
1200
1000
800
600
400
200
0
mE (422nm)
0
500
1000
1500
2000
B 12 (pg/ml)
①
②